# EUROPEAN PATENT SPECIFICATION

(11) **EP 1 294 381 B1**
(45) Date of publication and mention of the grant of the patent: **31.08.2005**
(21) Application number: 01945490.9
(22) Date of filing: 29.06.2001
(51) Int. Cl.: A61K 31/56

(54) **NEUROPROTECTIVE 7-BETA-HYDROXYSTEROIDS**
NEUROPROTEKTIVE 7-BETA-HYDROXYSTEROIDE
7-BETA-HYDROXYSTEROIDES NEUROPROTECTEURS

(30) Priority: 29.06.2000 GB 0016027
(43) Date of publication of application: 26.03.2003
(73) Proprietor: Hunter-Fleming Limited, Dorset SP7 9ND (GB)
(72) Inventor: WÜLFERT, Ernst, B-1180 Brussels (BE); PRINGLE, Ashley Ker, Southampton, Hampshire SO19 7JF (GB); SUNDSTROM, Lars Eric, Old Alresford, Hampshire SO24 9DU (GB)
(74) Representative: Tubby, David George
(86) International application number: PCT/GB2001/002929
(87) International publication number: WO 2002/000224

(56) References cited:
- WO-A-01/32680
- WO-A-01/60375
- WO-A-94/03176
- WO-A-97/37664
- WO-A-98/08868
- WO-A-99/15179
- WO-A-99/24400
- US-A- 5 925 630
- US-A- 5 985 242
- C. M. P. RODRIGUES ET AL.: "Amyloid .beta.-Peptide Disrupts Mitochondrial Membrane Lipid and Protein Structure: Protective Role of Tauroursodeoxycholate" BIOCHEM. BIOPHYS. RES. COMMUN., vol. 281, no. 2, 2001, pages 468-474, XP001019285

## Description

The present invention relates to the use of a series of 3-hydroxy-7β-hydroxysteroid compounds and certain ketone derivatives thereof for protection against neuronal cell death, and which are thus useful in the treatment and prevention of such conditions or the sequelae of such conditions as Alzheimer's Disease, Parkinson's Disease, Cognitive Impairment No Dementia (CIND), stroke, brain trauma, spinal cord injury and peripheral nerve injury; they are also useful for enhancing cognitive function.

The production of 7α-hydroxylated metabolites of dehydroepiandrosterone (DHEA) *in vivo* has been known since 1959 with the identification of 7α-hydroxy-DHEA in urine [J J Schneider, M L Lewbart, Recent Progr. Horm. Res. 15 (1959) 201-230; L Starka *et al*, Clin. Chim. Acta. 7 (1961) 309-316)]. Since then, extensive 7α-hydroxylation of 3β-hydroxysteroid substrates (including DHEA and epiandrosterone - EPIA) has been reported in tissue preparations from many human organs, including adult and foetal liver, testes, epididymus, skin, mammary tissue, prostate, adipose stromal cells and tonsils. Hydroxylation of DHEA at the 7-position has also been demonstrated in rat liver and in numerous mouse tissues and organs. However, little or no attention has been paid to the 7β-equivalent. In all these studies, 7α-hydroxy-DHEA was by far the major metabolite produced. Indeed, Doostzadeh *et al* [Steroids 63 (1998) 608-614] reported that the production rate of 7α-hydroxy-DHEA by mouse liver microsomes was more than fifteen times the production rate of 7β-hydroxy-DHEA.

EPIA, DHEA and pregnenolone have also been shown to be rapidly and extensively transformed to their corresponding 7α-hydroxy metabolites in the rat brain [J M Guiraud *et al*, Steroids 34 (1979) 241-248; M Warner *et al*, Endocrinology 124 (1989) 2699-2706; Y Akwa *et al*, Biochem. J. 288 (1992) 959-964)].

WO97/37664 discloses the use of a variety of compounds, including 7α-hydroxy-substituted steroids, to treat neuropsychiatric, immune or endocrine disorders. Among the disorders suggested in WO97/37664 that these compounds may be used to treat is included Alzheimer's Disease. However, the mechanism suggested for this action is that the disorder is hypothesised to result from a deficit of the 7α-hydroxy-substituted steroid in the brain, and the treatment proposed in WO97/37664 thus rectifies this deficit by the administration of a 7α-hydroxy-substituted steroid to replace the missing compound. The procedure described in WO97/37664 thus treats an existing condition, rather than preventing the condition or preventing a worsening of the condition by preventing further neuronal damage. WO97/37664 does not, therefore, describe a neuroprotective effect. It is also predicated upon the belief that the active agent is the 7α compound, and that the 7β compound, if present, is inactive.

WO94/20111 also discloses the use of a number of DHEA derivatives for preventing or reducing the loss of tissue viability caused by adhesion of neutrophils to endothelial cells. However, this is not the mechanism by which the disorders treated by the present invention are caused.

Although the 7β-hydroxy analogues of the compounds disclosed in WO97/37664 were known to be produced *in vivo,* they are produced in amounts of less than 5%, as compared with more than 95% of the 7α-isomer. Furthermore, no enzyme system responible for the conversion of these 3-hydroxy steroids to their corresponding 7β-hydroxy derivatives has been characterised. For all these reasons and in the light of the research summarised above, it is clear from the literature that the general expectation was that the 7β-isomer would be inactive. As a result, as is clear from the literature summarised above, and a great deal more, essentially no investigation of possible biological activity of the 7β compounds has been carried out.

Contrary to this expectation, we have surprisingly found that the 7β-hydroxy-substituted steroids do have a biological activity, and that this activity is not the activity as described in WO97/37664 for the 7α-hydroxy-substituted steroids. Rather it is a neuroprotective activity such as has previously been demonstrated, albeit in a different class of compounds, in WO99/31049.

In events such as prolonged hypoxia and ischaemia, which may or may not be associated with hypoglycaemia, neuronal damage, to varying degrees, is encountered.

Ischaemia typically occurs during heart attacks, but the damage incurred at these times is substantially limited to the heart tissues, and certain treatments have been developed. With regard to the present invention, we are concerned the effects of both short term and more long term ischaemia on the brain, such as occurs with stroke patients or as a result of head injury, and also in more slowly developing neurodegenerative diseases in ageing where chronic sub-threshold levels of ischaemia and/or compromised energy supply may contribute to the brain degenerative changes observed. The severity of the ischaemia depends on the nature of the stroke or injury, but, invariably, there is brain damage, and it is this which the present invention addresses.

Various neuroprotective agents are known in the art which attempt to alleviate the problem of brain damage, but all of those currently known tend to be associated with adverse side effects. For example, MK801 (dizocilpine maleate) is a fairly simple molecule and is known to provide a level of neuroprotection to ischaemic patients. However, MK801 is also associated with "alarming psychotropic effects" (Martindale), as well as adverse motor effects. The neuroprotective effects are detailed in Brain Research 755 (1997) 36-46 (Pringle, A.K., *et al*), incorporated herein by reference. These same authors also described the neuroprotective effects of conotoxin in an earlier paper but, despite the neuroprotective effects of this compound, adverse side effects, *in vivo*, are observed.

Thus, the present invention consists in the use for the manufacture of a medicament for protection against neuronal damage of a 7β-hydroxy steroid or its pharmaceutically acceptable ester or other derivative thereof, according to formula (I) or (II): 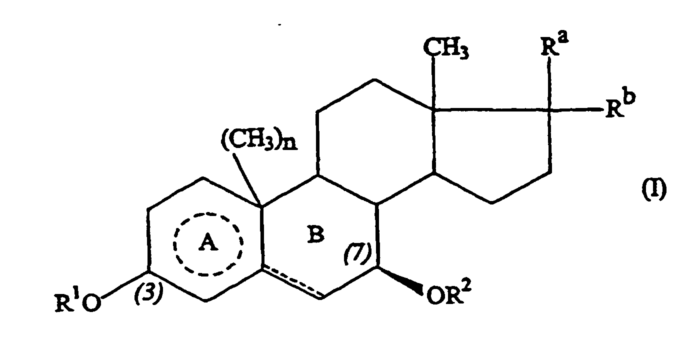 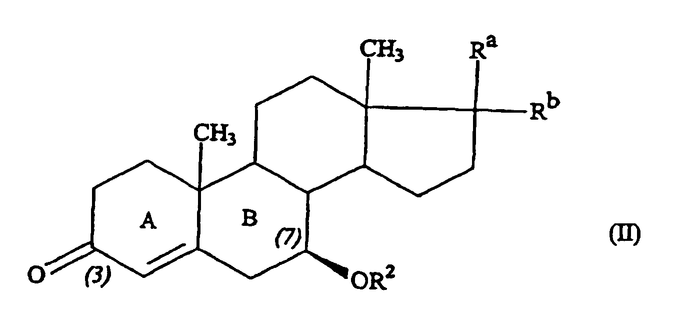 wherein
R¹ and R² are the same as or different from each other and each represents a hydrogen atom, an alkyl group having from 1 to 6 carbon atoms, an alkenyl group having from 2 to 6 carbon atoms, an alkynyl group having from 2 to 6 carbon atoms, an aryl group having from 6 to 10 carbon atoms, a formyl group, an alkylcarbonyl group having from 2 to 7 carbon atoms, an alkenylcarbonyl group having from 3 to 7 carbon atoms, an alkynylcarbonyl group having from 3 to 7 carbon atoms, an arylcarbonyl group having from 7 to 11 carbon atoms, an aralkylcarbonyl group having from 8 to 15 carbon atoms, an aralkenylcarbonyl group having from 9 to 15 carbon atoms, or a heterocyclic-carbonyl group, as defined below;
one of R^{a} and R^{b} represents a hydroxy group, preferably in the β configuration, and the other represents a hydrogen atom, or R^{a} and R^{b} together represent an oxo group;
the ring A, 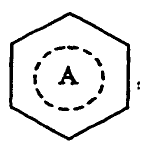 is a benzene or cyclohexane ring;
when ring A is a cyclohexane ring, the dotted line in ring B represents a single or double carbon-carbon bond and n is 1; or when ring A is a benzene ring, the dotted line in ring B represents a single carbon-carbon bond and n is 0;
said heterocyclic-carbonyl group is a group of formula R³-CO, where R³ represents a heterocyclic group having from 3 to 7 ring atoms, of which from 1 to 3 are hetero-atoms selected from nitrogen atoms, oxygen atoms and sulphur atoms, and the remaining atom or atoms of which there is at least one is or are carbon atoms;
said alkyl, alkenyl and alkynyl groups and the alkyl, alkenyl and alkynyl parts of said alkylcarbonyl, alkenylcarbonyl and alkynylcarbonyl groups being unsubstituted or having at least one of the following substituents ψ:
substituents ψ: hydroxy groups, mercapto groups, halogen atoms, amino groups, alkylamino groups having from 1 to 6 carbon atoms, dialkylamino groups in which each alkyl group has from 1 to 6 carbon atoms, carbamoyl groups, nitro groups, alkoxy groups having from 1 to 6 carbon atoms, alkylthio groups having from 1 to 6 carbon atoms, carboxy groups, alkoxycarbonyl groups and unsubstituted aryl groups having from 6 to 10 carbon atoms;
said aryl groups, said heterocyclic groups, and the aryl parts of said arylcarbonyl groups and said aralkylcarbonyl groups being unsubstituted or having at least one of the following substituents ξ:
   substituents ξ: any of substituents ψ, and alkyl groups having from 1 to 6 carbon atoms, hydroxyalkyl groups having from 1 to 6 carbon atoms, and haloalkyl groups having from 1 to 6 carbon atoms;
and pharmaceutically acceptable salts and esters thereof.

A particular class of 7β-hydroxy steroids which are of especial interest to the present invention are the 3β,7β-dihydroxy steroids and pharmaceutically acceptable esters thereof.

Preferred esters are carboxylic acid esters.

More preferably, in the compounds of formula (I):
R¹ and R² are the same as or different from each other and each represents a hydrogen atom, an alkyl group having from 1 to 6 carbon atoms, an optionally substituted phenyl group, a formyl group, an alkylcarbonyl group having from 2 to 5 carbon atoms, an arylcarbonyl group having from 7 to 11 carbon atoms, an aralkylcarbonyl group having from 8 to 15 carbon atoms, or a heterocyclic-carbonyl group, as defined below;
one of R^{a} and R^{b} represents a hydroxy group in the β configuration, and the other represents a hydrogen atom, or R^{a} and R^{b} together represent an oxo group;
said heterocyclic-carbonyl group is a group of formula R³-CO , where R³ represents a heterocyclic group having from 3 to 7 ring atoms, of which from 1 to 3 are hetero-atoms selected from nitrogen atoms, oxygen atoms and sulphur atoms, and the remaining atom or atoms of which there is at least one is or are carbon atoms.

In the compounds of the present invention, where R¹, R², or substituent ξ is an alkyl group, this may be a straight or branched chain alkyl group having from 1 to 6 carbon atoms, and examples include the methyl, ethyl, propyl, isopropyl, butyl, isobutyl, sec-butyl, t-butyl, pentyl, 1-methylbutyl, 2-methylbutyl, 3-methylbutyl, 1-ethylpropyl, 2-ethylpropyl, 1,1-dimethylpropyl, hexyl, 1-methylpentyl, 2-methylpentyl, 3-methylpentyl, 4-methylpentyl, 1-ethylbutyl, 2-ethylbutyl, 3-ethylbutyl, t-hexyl, and 1,1-dimethylpeatyl groups, of which those groups having from 1 to 4 carbon atoms are preferred, the methyl and ethyl groups being most preferred.

Where R¹ or R² represents an alkenyl group, this may be a straight or branched chain alkenyl group having from 2 to 6 carbon atoms, and examples include the vinyl, 1-propenyl, allyl, isopropenyl, methallyl, 1-, 2-, 3-butenyl, isobutenyl, 1-, 2-, 3-, 4-pentenyl and 1-, 2-, 3-, 4-, 5-hexenyl groups, of which those alkenyl groups having from 2 to 4 carbon atoms are preferred, the vinyl and allyl groups being most preferred.

Where R¹ or R² represents an alkynyl group, this may be a straight or branched chain alkynyl group having from 2 to 6 carbon atoms, and examples include the ethynyl, 1-, 2-propynyl, 1-, 2-, 3-butynyl, isobutynyl, 1-, 2-, 3-, 4-pentynyl and 1-, 2-, 3-, 4-, 5-haxynyl groups, of which those alkynyl groups having from 2 to 4 carbon atoms are preferred.

Where R¹, R² or substituent ψ represents an aryl group, this is an aromatic carbocyclic group having from 6 to 10 carbon atoms. Examples of such groups include the phenyl, 1-naphthyl, 2-naphthyl and indenyl groups, of which the phenyl group is preferred. Except in the case of substituent α, these groups may be substituted or unsubstituted. Where the group is substituted, the number of substituents is limited only by the number of substitutable positions, and possibly, in some instances, by steric constraints. Thus, in the case of the phenyl groups, the maximum number of substituents is 5, in the case of the naphthyl groups, the maximum number of substituents is 7 and so on. However, a preferred number of substituents is from 1 to 3, and the substituents are as hereafter described.

Where R¹ or R² represents an alkylcarbonyl group, this is an alkanoyl group, which may be a straight or branched chain group having from 2 to 7 carbon atoms (i.e. from 1 to 6 carbon atoms in the alkyl part), and examples include the acetyl, propionyl, butyryl, isobutyryl, valeryl, isovaleryl, pivaloyl, hexanoyl, and heptanoyl groups, of which those groups having from 2 to 5 carbon atoms are preferred, the acetyl and propionyl groups being most preferred. The alkyl portion of this group may be substituted or unsubstituted, and, if substituted, the substituents are selected from substituents α. Examples of such substituted groups include the alanyl, β-alanyl, phenylalanyl, asparaginyl, cysteinyl, glycoloyl, glycyl, methionyl, omithyl, glyceroyl, tropoyl, glutaminyl, glutamyl, homocysteinyl, seryl, homoseryl, threonyl, lactoyl, leucyl, isoleucyl, norleucyl, lysyl, valyl, norvalyl and sarcosyl groups.

Where R¹ or R² represents an alkenylcarbonyl group, this may be a straight or branched chain alkenylcarbonyl group having from 3 to 7 carbon atoms, and examples include the acryloyl, methacryloyl, crotonoyl, isocrotonoyl, 3-butenoyl, pentenoyl and hexenoyl groups, of which those alkenylcarbonyl groups having from 3 to 5 carbon atoms are preferred, the acryloyl and methacryloyl groups being most preferred.

Where R¹ or R² represents an alkynylcarbonyl group, this may be a straight or branched chain alkynylcarbonyl group having from 3 to 7 carbon atoms, and examples include the propioloyl, 3-butynylcarbonyl, pentynylcarbonyl and hexynylcarbonyl groups, of which those alkynylcarbonyl groups having from 3 to 5 carbon atoms are preferred.

Where R¹ or R² represents an arylcarbonyl group, the aryl part of this may be any of the aryl groups defined and exemplified above. Preferred arylcarbonyl groups include the benzoyl, o-, m- or p-toluoyl, o-, m- or p-anisoyl, o-, m- or p-hydroxybenzoyl, picryl, galloyl, protocatechuoyl, vanilloyl, veratroyl, anthraniloyl, I-naphthoyl and 2-naphthoyl groups.

Where R¹ or R² represents an aralkylcarbonyl or aralkenylcarbonyl group, the aryl and, as the case may be, alkyl or alkenyl group may be any of those groups defined and exemplified above. Specific examples of such groups include the phenylacetyl, 3-phenylpropionyl, benziloyl, tyrosyl, atropoyl, hydratropoyl and cinnamoyl groups.

Where R¹ or R² represents a heterocyclic-carbonyl group, this is a group of formula R³-CO-, where R³ represents a heterocyclic group having from 3 to 7 ring atoms, of which from 1 to 3 are nitrogen, oxygen or sulphur atoms, the remainder being carbon atoms. At least one of the ring atoms should be a carbon atom. Where there are 3 hetero-atoms, it is preferred that at least one is a nitrogen atom. Examples of such groups include the 2- and 3-furoyl, 2- and 3-thenoyl, 2-pyridinecarbonyl, nicotinoyl, isonicotinoyl, prolyl, piperidinecarbonyl, piperazinecarbonyl and morpholinocarbonyl groups.

Where substituent ψ or substituent ξ is an alkylamino group having from 1 to 6 carbon atoms, the alkyl part may be any of the alkyl groups defined and exemplified above. Preferred examples of such alkylamino groups include the methylamino, ethylamino, propylamino, isopropylamino, butylamino, isobutylamino, sec-butylamino, t-butylamino, pentylamino, isopentylamino, neopentylamino, t-pentylamino, hexylamino, and isohexylamino groups, of which those groups having from 1 to 4 carbon atoms are preferred, the methylamino and ethylamino groups being most preferred.

Where substituent ψ or substituent ξ is a dialkylamino group, each alkyl part has from 1 to 6 carbon atoms, and the two alkyl groups may be the same as or different from each other. The alkyl groups may be any of the alkyl groups defined and exemplified above. Preferred examples of such dialkylamino groups include the dimethylamino, methylethylamino, diethylamino, methylpropylamino, dipropylamino, diisopropylamino, ethylbutylamino, dibutylamino, di-t-butylamino, methylpentylamino, dipentylamino, diisopentylamino, and dihexylamino groups, of which those groups having from 1 to 4 carbon atoms in each alkyl group are preferred, the dimethylamino and diethylamino groups being most preferred.

Where substituent ψ or substituent ξ is an alkoxy group, this may be a straight or branched chain alkoxy group having from 1 to 6 carbon atoms, and examples include the methoxy, ethoxy, propoxy, isopropoxy, butoxy, isobutoxy, sec-butoxy, t-butoxy, pentyloxy, isopentyloxy, neopentyloxy, t-pentyloxy, hexyloxy, and isohexyloxy groups, of which those groups having from 1 to 4 carbon atoms are preferred, the methoxy and ethoxy groups being most preferred.

Where substituent ψ or substituent ξ is an alkylthio group having from 1 to 6 carbon atoms, the alkyl part may be any of the alkyl groups defined and exemplified above. Preferred examples of such alkylthio groups include the methylthio, ethylthio, propylthio, isopmpylthio, butylthio, isobutylthio, sec-butylthio, t-butylthio, pentylthio, isopentylthio, neopentylthio, t-pentylthio, hexylthio, and isohexylthio groups, of which those groups having from 1 to 4 carbon atoms are preferred, the methylthio and ethylthio groups being most preferred.

Where substituent ψ or substituent ξ is an alkoxycarbonyl group, this may be a straight or branched chain alkoxycarbonyl group having from 2 to 7 carbon atoms, and examples include the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl, isopentyloxycarbonyl, neopentyloxycarbonyl, t-pentyloxycarbonyl, hexyloxycarbonyl, and isohexyloxycarbonyl groups, of which those groups having from 1 to 4 carbon atoms are preferred, the methoxycarbonyl and ethoxycarbonyl groups being most preferred.

Where substituent ξ is a hydroxyalkyl group having from 1 to 6 carbon atoms, the alkyl part may be any of the alkyl groups defined and exemplified above. Preferred examples of such hydroxyalkyl groups include the hydroxymethyl, 1- and 2-hydroxyethyl, 1-, 2- and 3-hydroxypropyl, 1,2-dihydroxyethyl, 1,2,3-trihydroxypropyl, 4-hydroxybutyl, 5-hydroxypentyl and 6-hydroxyhexyl groups.

Where substituent ξ is a haloalkyl group having from 1 to 6, preferably from 1 to 4, carbon atoms, the alkyl part may be as defined and exemplified above, and the halogen atom is preferably chlorine, fluorine, bromine or iodine. Examples of such groups include the fluoromethyl, chloromethyl, bromomethyl, iodomethyl, dichloromethyl, difluoromethyl, trichloromethyl, trifluoromethyl, 2,2,2-trichloroethyl, 2-chloroethyl, 2-fluoroethyl, 2-bromoethyl, 2-iodoethyl, 2,2-dibromoethyl, 2,2,2-tribromoethyl, 3-fluoropropyl, 3-chloropropyl, 4-bromobutyl, 4-fluorobutyl, 5-fluoropentyl and 6-fluorohexyl groups.

It will be appreciated that, where the compound contains a group of formula -OR, where R is any of the groups and atoms defined above in relation to R¹ etc., the active species is likely to be the compound containing the free hydroxy group. Accordingly, any group that can be converted *in vivo* to a hydroxy group may be used in place of the hydroxy group.

Specific examples of compounds of the present invention include:

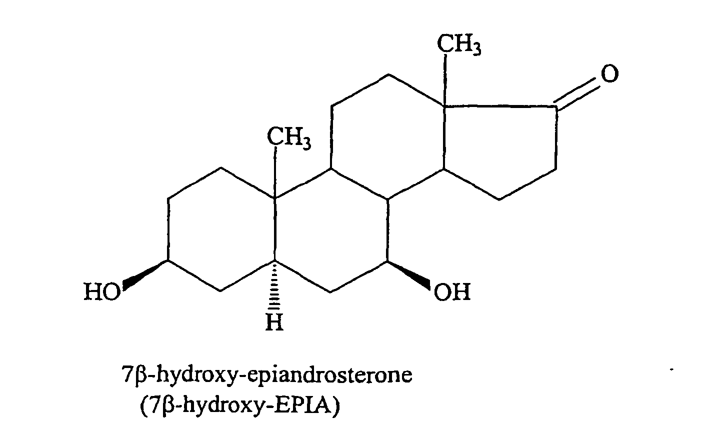

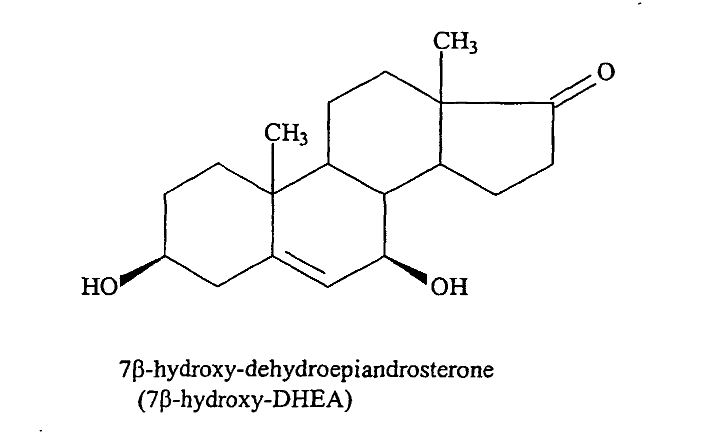

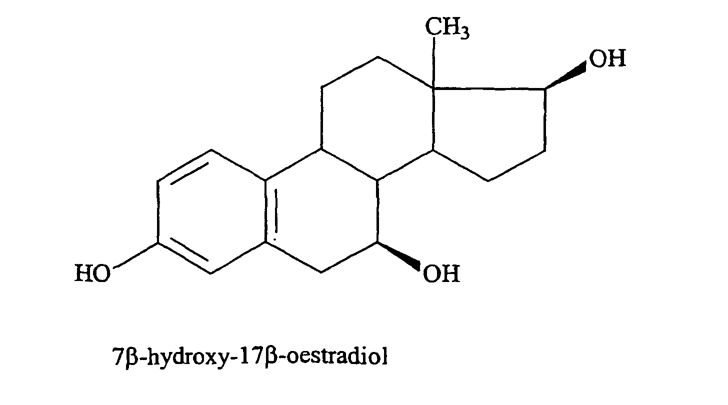

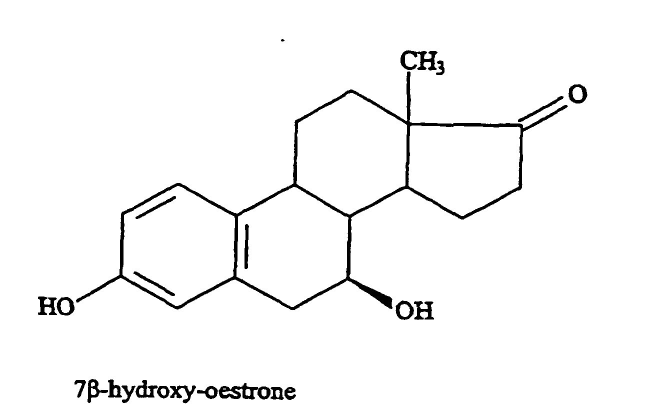

In addition, the following 7α-hydroxy compound is thought to be active in the same way:

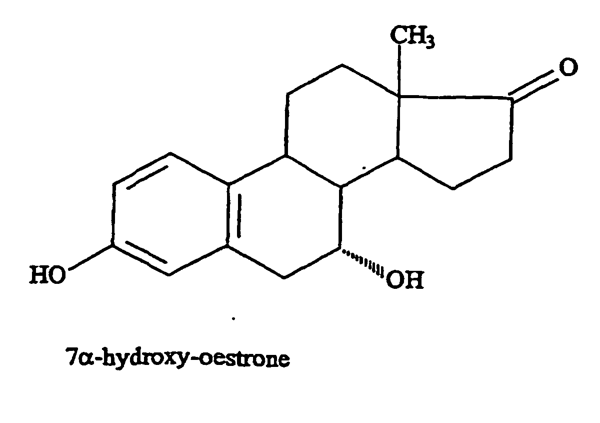

We have surprisingly discovered that these compounds can be used to protect against acute and chronic neuronal damage caused by such events as stroke, brain trauma and cerebral ischaemia such as may be induced by sub-arachnoid haemhorrage or which occurs during heart bypass surgery etc.

The compounds of the present invention may be prepared by a variety of processes, well known in themselves, starting from the parent steroids. For example, they may be prepared by the methods described in the literature referred to above, which would give a mixture of the 7β and corresponding 7α compounds, which may then be separated by well known techniques.

As an example, 7β-hydroxy EPIA may be obtained from DHEA by allylic oxidation after protection of the 3β-hydroxy group and the 17-ketone group using conventional methods. The product is then reduced with a soluble metal compound catalyst (such as sodium hydride) and the 3β-hydroxy and 17-ketone groups are deprotected. The 7α-hydroxy and 7β-hydroxy epimers may then be separated by conventional means, for example column chromatography, and the 7β-hydroxy EPIA may be crystallised to purity.

An alternative synthetic method is shown in the following reaction scheme:

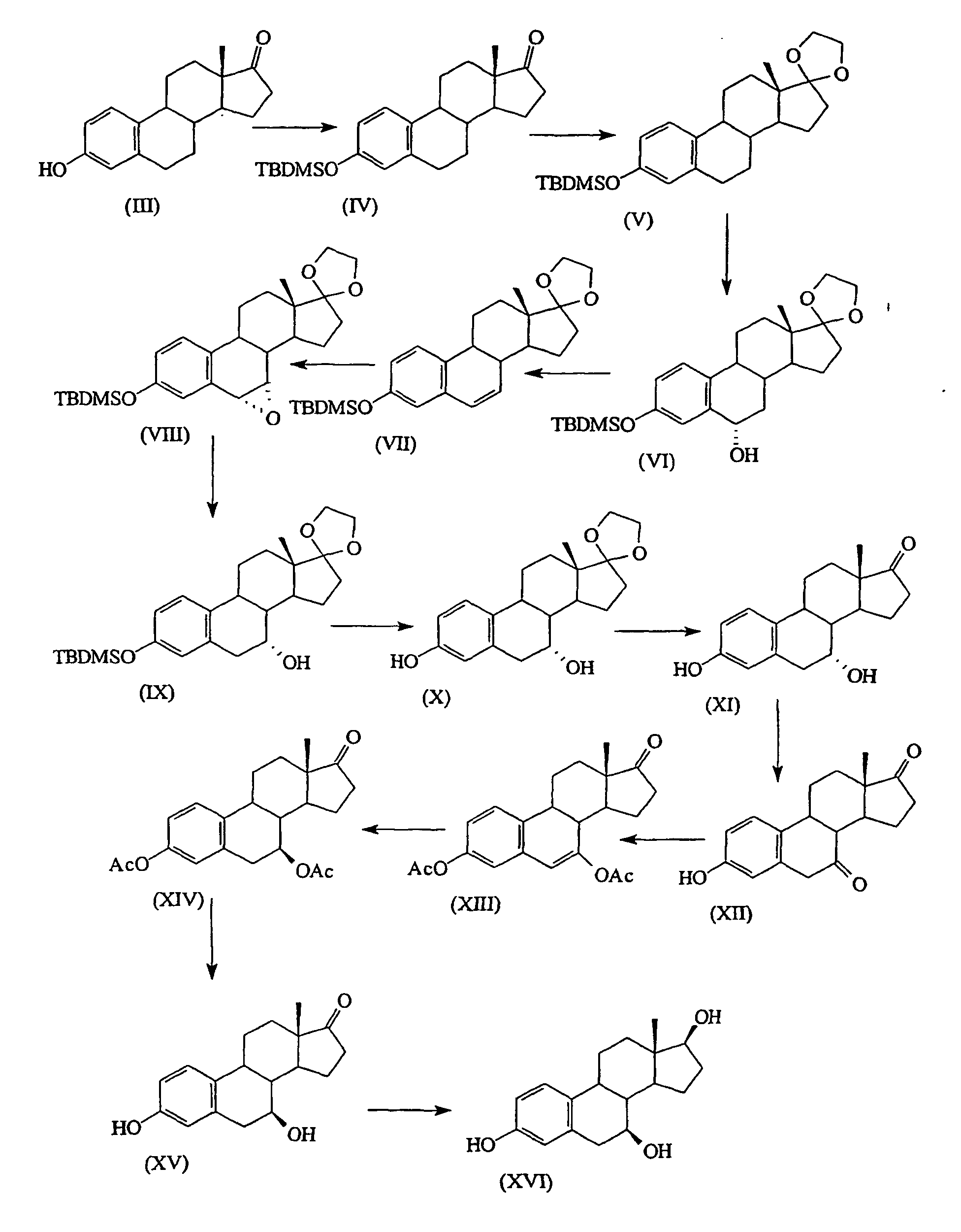

In the above formulae, TBDMSO represents t-butyldimethylsilyloxy and Ac represents acetyl.

In the first step of the above reaction scheme, the compound of formula (III), oestrone, is protected by a t-butyldimethylsilyloxy group in a conventional manner to give the protected compound of formula (IV). This is then reacted with ethylene glycol in the presence of an acid catalyst (such as p-toluenesulphonic acid) to protect the keto group at the 17 position and give the compound of formula (V). A hydroxy group may then be introduced at the 6-position as illustrated hereafter in Example 3, to give the compound of formula (VI), which is then dehydrated to give the compound of formula (VII). This is epoxidised to give the compound of formula (VIII), which is then reduced to the compound of formula (IX), with a 7α-hydroxy group. The t-butyldimethylsilyl protecting group is removed, giving the compound of formula (X), and this is heated with a catalytic amount of an acid, to give 7α-hydroxy-oestrone (XI). This is oxidised, e.g. using chromic acid/sulphuric acid, to give the 7-keto-oestrone (XII), which is then reacted with acetic anhydride, to give the compound of formula (XIII). This compound is hydrogenated, e.g. using hydrogen in the presence of a palladium catalyst, to give the compound of formula (XIV), and finally the acetyl groups are removed, to give 7β-hydroxy-oestrone (XV), a compound of the present invention. If desired, this may be reduced, to give 7β-hydroxy-oestradiol (XVI), also a compound of the present invention.

Other 7β-hydroxy- compounds of the present invention may be prepared in a similar manner, for example, 7β-hydroxy-DHEA may be prepared as illustrated by the following reaction scheme:

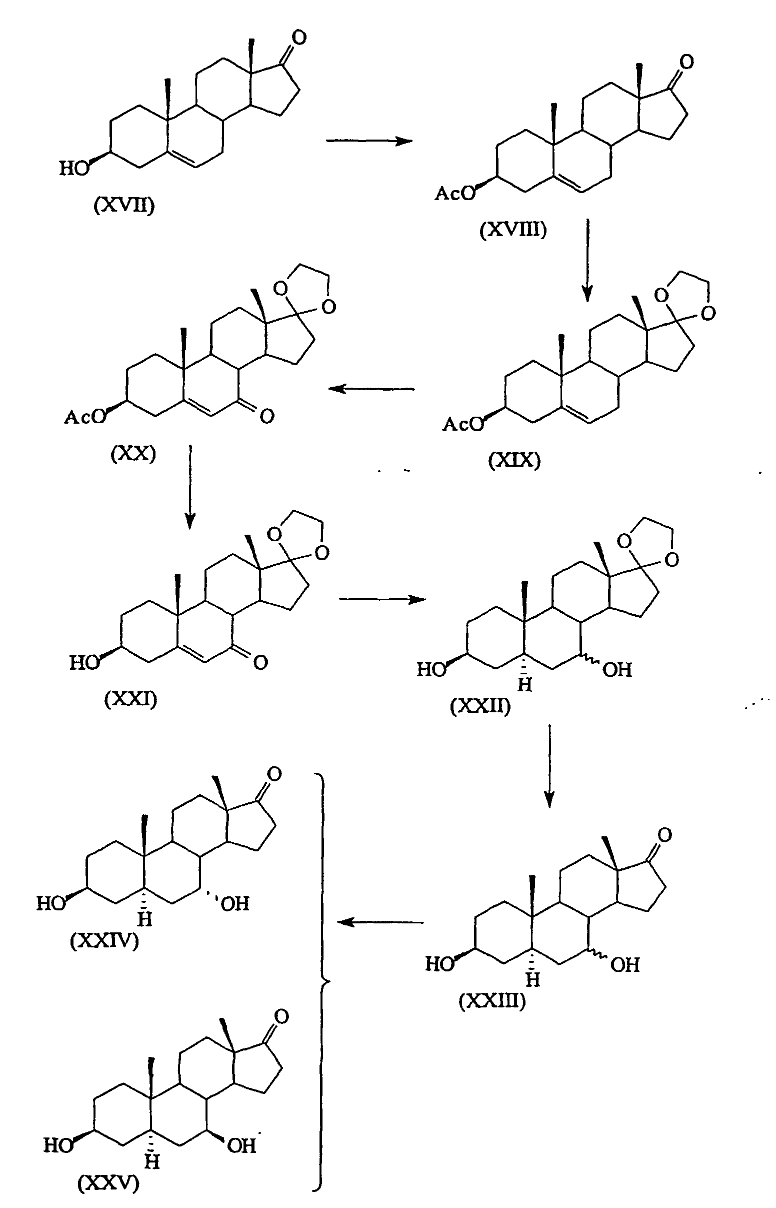

In this reaction scheme, DHEA (XVII) is acetylated to give the corresponding acetate of formula (XVIII), which is then reacted with ethylene glycol, to give the ketal of formula (XIX). The ketal (XIX) is then oxidised as described in Example 16, to give the corresponding 7-keto compound (XX), which is then deacetylated, to give the compound of formula (XXI). This is reduced, to give 7-hydroxy-17-ketal-EPIA of formula (XXII), which is then treated with an acid to remove the ketal group and give 7-hydroxy-EPIA, which is finally separated into the 7β- and 7α- isomers by chromatography, to give 7α-hydroxy-EPIA (XXIV) and 7β-hydroxy-EPIA (XXV).

The compounds of the present invention may be applied to the patient if it is suspected that they are in danger of an ischaemic event, especially a stroke or head injury. Such prophylactic application may be exceedingly useful. However, it has also been demonstrated that the compounds of the present invention have useful activity, even if applied after an ischaemic event, but it will be appreciated that it is preferred to administer the compounds as soon as possible, in order to avoid as much neuronal degeneration as possible. In some circumstances it may be desirable to administer repeated doses, especially where the patient remains in danger of an ischaemic event.

Suitable methods of administration are generally by injection, in order to achieve the desired result as soon as possible. Thus, intravenous injection is particularly preferred but, in some circumstances it may be preferable to administer the compound directly into the cerebrospinal fluid.

The dose of the compound of the present invention will vary depending upon many factors, including the age, body weight and general condition of the patient, as well as the mode, frequency and route of administration. However, a dose of from 0.01 to 50 mg/kg body weight is generally recommended, a dose of from 0.05 to 20 mg/kg body weight being more preferred. This may be administered in a single dose or in divided doses.

The invention is further illustrated by the following non-limiting Examples, of which Examples 1 to 20 illustrate the preparation of compounds of the present invention and Examples 21 and 22 illustrate their activity. In Examples 1 to20, the Roman numerals refer to the formulae in the reaction schemes shown above.

### EXAMPLE 1

### 3-t-Butyldimethylsilyl-oestrone (IV)

4.25 g of t-butyldimethylsilyl chloride (28.2 mmol, 3 eq.) were added to a solution of 50 ml of dimethylformamide (DMF) containing 2.54 g of oestrone (III) (9.41 mmol, 1 eq.) and 3.84 g of imidazole (56.5 mmol, 6 eq.) in a 100 ml three-necked flask. The mixture was then left overnight at room temperature under a nitrogen atmosphere. A 10% w/v aqueous potassium carbonate solution was added to the reaction medium, which was then extracted with ethyl acetate. The organic phase was washed with water and then dried over anhydrous sodium sulphate and evaporated to dryness. 3.76 g of 3-t-butyldimethylsilyl-oestrone 2 (9.41 mmol, 100%) were obtained.

### EXAMPLE 2

### 17-Ketal-3-t-butyldimethylsilyl-oestrone (V)

A solution of 60 ml of toluene containing 3 g of 3-t-butyldimethylsilyl-oestrone (IV) (7.50 mmol), 3 ml of ethylene glycol and a catalytic amount of p-toluenesulphonic acid was heated to reflux with steam distillation using a Dean-Stark apparatus for 24 hours. The reaction medium was then poured into 50 ml of a 10% w/v aqueous potassium carbonate solution. The organic phase was decanted. The aqueous phase was extracted with ethyl acetate. The organic phases were combined and evaporated to dryness. 3.16 g of 17-ketal-3-t-butyldimethylsilyl-oestrone (V) (7.12 mmol, 95%) were obtained.

### EXAMPLE 3

### 6α-Hydroxy-17-ketal-3-t-butyldimethylsilyl-oestrone (VI)

In a 11 three-necked flask, a solution of 100 ml of anhydrous tetrahyrofuran (THF) was degassed by nitrogen flushing and cooled to -80°C. Diisopropylamine (20 ml, 143.30 mmol) was added to the reaction medium. A 15% w/v butyl lithium solution in cyclohexane (89.9 ml, 143.30 mmol) was added dropwise to the reaction medium. After 10 minutes, a solution of 100 ml of anhydrous THF, previously degassed, containing 17.5 g of potassium t-butylate was added dropwise to the reaction medium. After a further 15 minutes, a solution of 50 ml of anhydrous THF, previously degassed, containing 12.27 g of 17-ketal-3-t-butyldimethylsilyl-oestrone (V) (27.63 mmol) was added dropwise to the reaction medium. The reaction mixture was left for 2 hours at -80°C. At the end of this time, 48 ml of trimethyl borate (429.90 mmol) were added dropwise at -80°C to the reaction medium, which was left at 0°C for 1 hour. 100 ml of 30% v/v aqueous hydrogen peroxide solution were then added. The reaction mixture was left for 1 hour at room temperature and then 500 ml of water were added. The reaction medium was extracted with ethyl acetate. The organic phase was washed with a 10% w/v aqueous sodium thiosulphate solution, washed with water, dried over anhydrous sodium sulphate and evaporated to dryness. The residue was purified by flash chromatography (SiO2/ethyl acetate : cyclohexane 1/9 then 2/8). 6.35 g of 6α-hydroxy-17-ketal-3-t-butyldimethylsilyl-oestrone 4 (13.81 mmol, 50%) were obtained.

### EXAMPLE 4

### 17-Ketal-3-tert-butyldimethylsilyl-6-dehydroestrone (VII)

A solution of 40 ml of toluene containing 1.54 g of 6α-hydroxy-17-ketal-3-t-butyldimethylsilyl-oestrone (VI) (3.35 mmol), 4 ml of ethylene glycol and a catalytic amount of p-toluenesulphonic acid was heated to reflux with steam distillation using a Dean-Stark apparatus for 24 hours. The reaction medium was the npoured into 50 ml of a 10% w/v aqueous potassium carbonate solution. The organic phase was decanted. The aqueous phase was then extracted with ethyl acetate. The organic phases were combined and evaporated to dryness. 1.48 g of 17-ketal-3-t-butyldimethylsilyl-6-dehydroestrone (VII) (3.35 mmol, 100%) were obtained.

### EXAMPLE 5

### 17-Ketal-3-tert-butyldimethylsilyl-6α,7α-epoxyoestrone (VIII)

A solution of 20 ml of dichloromethane containing 1.16 g of m-chlorobenzoic acid (55%, 3.69 mmol, 1.1 eq.) was added dropwise, at 0°C, to a solution of 20 ml of dichloromethane containing 1.85 g of 17-ketal-3-t-butyldimethylsilyl-6-dehydroestrone (VII) (3.36 mmol, 1 eq.). The reaction medium was poured, after 2 hours, into a 10% w/v aqueous sodium hydrogen carbonate solution and then extracted with ethyl acetate. The organic phase was dried over anhydrous sodium sulphate and then evaporated to dryness. The residue was purified by flash chromatography (SiO2/ethyl acetate : cyclohexane 1/9). 769 mg of 17-ketal-3-t-butyldimethylsilyl-6α,7α-epoxyoestrone 6 (1.68 mmol, 50%) were obtained.

### EXAMPLE 6

### 7α-Hydroxy-17-ketal-3-tert-butyldimethylsilyl-oestrone (IX)

200 mg of lithium aluminium hydride (5.40 mmol, 2 eq.) were added to a solution of 50 ml of anhydrous THF containing 1.13 g of 17-ketal-3-t-butyldimethylsilyl-6α,7α-epoxyoestrone 6 (2.60 mmol, 1 eq.). The reaction medium was heated to reflux for 2 hours and then cooled, poured into ice, filtered through a Celite (trade mark) filter aid and extracted with ethyl acetate. The organic phase was dried over anhydrous sodium sulphate and then evaporated to dryness. The residue was purified by flash chromatography (SiO2/ethyl acetate : cyclohexane 1/9). 837 mg of 7α-hydroxy-17-ketal-3-t-butyldimethylsilyl-oestrone (IX) (1.82 mmol, 70%) were obtained.

### EXAMPLE 7

### 7α-Hydroxy-17-ketal-oestrone (X)

A solution of 20 ml of THF containing 1.5 g of tetrabutylammonium chloride (4.78 mmol, 1.10 eq.) was added, at room temperature, to a solution of 50 ml of THF containing 2 g of 7α-hydroxy-17-ketal-3-t-butyldimethylsilyl-oestrone (IX) (4.35 mmol, 1 eq.). The reaction medium was poured into 70 ml of a 10% w/v aqueous sodium carbonate solution. The reaction medium was extracted with ethyl acetate. The organic phase was dried over anhydrous sodium sulphate and then evaporated to dryness. 1.39 g of 7α-hydroxy-17-ketal-oestrone (X) (4.22 mmol, 97%) were obtained.

### EXAMPLE 8

### 7α-Hydroxy-oestrone (XI)

A solution of 50 ml of acetone containing 1 ml of water, 1.0 g of 7α-hydroxy 17-ketal-oestrone (X) (3.03 mmol) and a catalytic amount of p-toluenesulphonic acid was heated to reflux for 2 hours. The reaction medium was the npoured into 70 ml of a 10% w/v aqueous sodium carbonate solution. The reaction medium was extracted with ethyl acetate. The organic phase was dried over anhydrous sodium sulphate and then evaporated to dryness. 814 mg of 7α-hydroxy-oestrone (XI) (2.85 mmol, 94%), which was recrystallised from ethyl acetate, were obtained.

### EXAMPLE 9

### 7-Ketoestrone (XII)

An 8 N solution of chromic acid in sulphuric acid was added dropwise, until the yellow colour persisted, to a solution cooled to 0°C of 40 ml of acetone containing 300 mg of 7α-hydroxy-oestrone (XI) (1.05 mmol). The reaction medium was poured into 50 ml of water and then extracted with ethyl acetate. The organic phase was washed with an aqueous sodium carbonate solution and then dried over anhydrous sodium sulphate and evaporated to dryness. The residue was purified by flash chromatography (SiO2/ethyl acetate : cyclohexane 3/7). 200 mg of 7-keto-estrone 10 (0.70 mmol, 67%) were obtained.

### EXAMPLE 10

### 7-Hydroxy-6-dehydroestrone 3,7-diacetate (XIII)

A solution of 10 ml of acetic anhydride containing 5 g of anhydrous sodium acetate and 1 g of 7-keto-oestrone (XII) (3.52 mmol) was heated to reflux for 1 hour. The reaction medium was then cooled and then poured into water and extracted with diethyl ether. The organic phase was washed with an aqueous sodium carbonate solution and then dried over anhydrous sodium sulphate and evaporated to dryness. The residue was purified by flash chromatography (SiO2/ethyl acetate) : cyclohexane 1/9). 1.25 g of 7-hydroxy-6-dehydroestrone-3,7-diacetate (XIII) (3.41 mmol, 97%) were obtained.

### EXAMPLE 11

### 7-Hydroxyestrone 3,7-diacetate (XIV)

A solution of 80 ml of glacial acetic acid containing 1.0 g of 7-hydroxy-6-dehydroestrone-3,7-diacetate (XIII) (2.72 mmol) was hydrogenated with 200 mg of 10% palladium on charcoal catalyst under a hydrogen pressure of 1 bar. The reaction medium was filtered after 2 hours and evaporated to dryness. The residue was purified by flash chromatography (SiO2/ethyl acetate : cyclohexane 1/9). 855 mg of 7-hydroxyestrone 3,7-diacetate (XIV) (2.31 mmol, 85%) were obtained.

### EXAMPLE 12

### 7β-Hydroxyestrone (XV)

A solution of 50 ml of methanol containing 1% of potassium hydroxide and 1 g of 7-hydroxyestrone-3,7-diacetate 12 (2.70 mmol) was heated to reflux for 2 hours. The reaction medium was then cooled, neutralised and then extracted with ethyl acetate. The organic phase was dried over anhydrous sodium sulphate and then evaporated to dryness. 695 mg of 7β-hydroxyestrone (XV) (2.43 mmol, 90%), which was recrystallised from methanol, were obtained.

### EXAMPLE 13

### 7β-Hydroxyestradiol (XVI)

264 mg of sodium borohydride (7.00 mmol, 2 eq.) were added to a solution of 50 ml of methanol containing 1.0 g of 7β-hydroxyestrone 13 (3.50 mmol). The reaction medium was poured into water and extracted with ethyl acetate. The organic phase was dried over anhydrous sodium sulphate and then evaporated to dryness. 917 mg of 7β-hydroxyestradiol 14 (3.18 mmol, 91 %), which was recrystallised from methanol, were obtained.

### EXAMPLE 14

### DHEA-3-acetate (XVIII)

A solution of 50 ml of pyridine and 50 ml of acetic anhydride containing 10 g of DHEA (XVII) (34.72 mmol) was heated to reflux for 4 hours. The reaction medium was cooled, poured into water and extracted with ethyl acetate. The organic phase was dried over anhydrous sodium sulphate and evaporated to dryness. 11.0 g of DHEA-3-acetate (XVIII) (33.33 mmol, 96%), which was recrystallised from ethanol, were obtained.

### EXAMPLE 15

### 17-Ketal-DHEA-3-acetate (XIX)

A solution of 100 ml of toluene containing 5 g of DHEA-3-acetate (XVIII) (15.15 mmol), 5 ml of ethylene glycol and a catalytic amount of p-toluenesulphonic acid was heated to reflux with steam distillation using a Dean-Stark apparatus for 24 hours. The reaction medium was poured into 100 ml of a 10% w/v aqueous potassium carbonate solution. The organic phase was decanted. The aqueous phase was extracted with ethyl acetate. The organic phases were combined and evaporated to dryness. 5.10 g of 17-ketal-3-DHEA-acetate (XIX) (13.64 mmol, 90%), which was recrystallised from ethanol, were obtained.

### EXAMPLE 16

### 7-Keto-17-ketal-DHEA-3-acetate (XX)

A solution of 70 ml of pyridine containing 5 g of 17-ketal-DHEA-3-acetate (XIX) (13.37 mmol) and a catalytic amount of Bengal Rose was irradiated using a medium-pressure mercury vapour lamp with oxygen sparging. A catalytic amount of copper acetate was added to the reaction medium after 24 hours. The reaction medium, after 24 hours, was evaporated to dryness. The residue was purified by flash chromatography (SiO2/ethyl acetate : cyclohexane 3/7). 3.11 g of 7-keto-17-ketal-DHEA-3-acetate (XX) (8.02 mmol, 60%) were obtained.

### EXAMPLE 17

### 7-Keto-17-ketal-DHEA (XXI)

A solution of 50 ml of methanol containing 1% of potassium hydroxide and 1 g of7-keto-17-ketal-DHEA-3-acetate (XX) (2.58 mmol) was heated to reflux for 2 hours. The reaction medium was then cooled, neutralised and then extracted with ethyl acetate. The organic phase was dried over anhydrous sodium sulphate and then evaporated to dryness. 802 mg of 7-keto-17-ketal-DHEA 5 (2.32 mmol, 90%), which was recrystallised from methanol, were obtained.

### EXAMPLE 18

### 7-Hydroxy-17-ketal-EPIA (XXII)

10 g of 7-keto-17-ketal-DHEA (XXI) (28.90 mmol) were added to a liquid ammonia solution at -33°C containing 2.65 g of sodium. After 4 hours, ammonium chloride was added until the blue colour disappeared. 2.65 g of sodium were then added. After 4 hours, ammonium chloride was again added until the blue colour disappeared. Water was added and the ammonia was allowed to evaporate. The reaction medium was extracted with ethyl acetate. The organic phase was dried over anhydrous sodium sulphate and then evaporated to dryness. 6.07 g of 7-hydroxy-17-ketal-EPIA (XXII) (17.34 mmol, 60%) were obtained.

### EXAMPLE 19

### 7-Hydroxy-EPIA (XXIII)

A solution of 100 ml of acetone containing 5 ml of water, 10 g of 7-hydroxy-17-ketal-EPIA (XXII) (28.57 mmol, 50%) and a catalytic amount of paratoluenesulphonic acid was heated to reflux for 4 hours. The reaction medium was cooled, poured into 100 ml of a 10% w/v aqueous sodium carbonate solution and then extracted with ethyl acetate. The organic phase was dried over anhydrous sodium sulphate and then evaporated to dryness. The residue was purified by flash chromatography (SiO₂/ethyl acetate). 5.24 g of 7-hydroxy-EPIA (XXIII) (17.14 mmol, 60%) were obtained.

### EXAMPLE 20

### 7α-Hydroxy-EPIA (XXIV) & 7β-hydroxy-EPIA (XXV)

7-Hydroxy-EPIA (XXIII) (5 g) containing 7α and 7β epimers in a ratio 65/35 was purified by flash chromatography (Al₂O₃/CHCl₃). 7β-Hydroxy-EPIA (XXV) (2.5 g) was obtained first, before 7α-hydroxy-EPIA (XXIV) (1.34 g). 7β-Hydroxy-EPIA (XXV) and 7α-hydroxy-EPIA (XXIV) were recrystallised from ethyl acetate.

### EXAMPLE 21

### Protocol For Studying Hypoxic Neuronal Damage

Organotypic hippocampal slice cultures were prepared using the basic method of Pringle *et al* (1996, 1997) modified as follows:

Wistar rat pups (8-11 days old) were decapitated and the hippocampus rapidly dissected into ice-cold Gey's balanced salt solution supplemented with 4.5mg/ml glucose. Slices were separated and plated onto Millicell CM culture inserts (4 per well) and maintained at 37°C/5% CO₂ for 14 days. Maintenance medium consisted of 25% heat-inactivated horse serum, 25% Hank's balanced salt solution (HBSS) and 50% minimum essential medium with added Earle's salts (MEM) supplemented with 1mM glutamine and 4.5mg/ml glucose. Medium was changed every 3-4 days.

Experimental hypoxia was performed as described previously (Pringle *et al*., 1996; 1997). Briefly, cultures were transferred to serum free medium (SFM - 75% MEM, 25% HBSS supplemented with 1mM glutamine and 4.5mg/ml glucose) containing 5µg/ml of the fluorescent exclusion dye propidium iodide (PI). Cultures were allowed to equilibrate in SFM for 60 minutes prior to imaging. PI fluorescence was detected using a Leica inverted microscope fitted with a rhodamine filter set. Any cultures in which PI fluorescence was detected at this stage were excluded from further study. Hypoxia was induced by transferring cultures to SFM (+PI) which had been saturated with 95%N₂/5%CO₂. Culture plates (without lids) were then sealed into an airtight chamber in which the atmosphere was saturated with 95%N₂/5%CO₂ by continuously blowing through gas at 10L/min for ten minutes before being sealed and placed in the incubator for 170mins (total time of hypoxia was therefore 180 mins). At the end of the hypoxic period cultures were returned to normoxic SFM containing PI and placed back in the incubator for 24 hours.

Neuronal damage was assessed as described previously (Pringle *et al*., 1996; 1997) using either NIH Image 1.60 running on an Apple IIsi computer or OpenLab 2.1 (Improvision) running on a Macintosh G4/400. Images were captured using a monochrome camera and saved onto optical disk for offline analysis. Light transmission images were captured prior to the addition of drugs, and PI fluorescence images recorded at the end of the 24-hour post-hypoxia recovery period. The area of the CA1 cell layer was determined from the transmission image. The area of PI fluorescence in CA1 was measured using the density slice function within NIH Image or OpenLab, and neuronal damage expressed as the percentage of the CA1 in which PI fluorescence was detected above background.

Steroid compounds were prepared by making an initial 1mg/ml solution in ethanol and further diluting down in SFM. Compounds were added to the cultures for 45 minutes prior to hypoxia, during the hypoxic episode and during the post-hypoxic recovery period. Control experiments consisted of cultures treated with vehicle alone.

### RESULTS

### Experiment 1:

An initial experiment was performed to determine whether 7αOH-EPIA and 7βOH-EPIA were neuroprotective at a high concentration of 100nM. Hypoxia produced a lesion in 25.5±6.4% of CA1. This damage was significantly reduced by both 7αOH-EPIA and 7βOH-EPIA when present pre-, during and post-hypoxia (see table I).

**Table I**

| Compound | N | % Damage in CA1 |
|---|---|---|
| Control Hypoxia | 17 | 25.5±6.4 |
| Hypoxia + 100nM 7αOH-EPIA | 16 | 4.0±2.9** |
| Hypoxia + 100nM 7βOH-EPIA | 16 | 9.0±4.7* |

### Experiment 2:

Having determined that both the α- and β-isomers of 7OH-EPIA were neuroprotective, we assessed the concentration-dependency of this effect. Control hypoxia resulted in neuronal damage to 31.9±4.7% of the CA1. 7βOH-EPIA was significantly neuroprotective at 10nM and 100nM, but activity was lost if the concentration was reduced to 1nM. as shown in Table II, below.

**Table II**

| Compound | N | % Damage in CA1 |
|---|---|---|
| Control Hypoxia | 29 | 31.9±4.7 |
| Hypoxia + 1nM 7βOH-EPIA | 15 | 20.6±7.2 |
| Hypoxia + 10nM 7βOH-EPIA | 12 | 11.9±4.7* |
| Hypoxia + 100nM 7βOH-EPIA | 13 | 14.3±5.0* |

### Experiment 3:

Having observed the neuroprotective activity of 7βOH-EPIA, we next investigated whether 7βOH-DHEA was neuroprotective. Cultures were incubated with either 100nM 7βOH-DHEA or vehicle, pre-, during and post-hypoxia. Hypoxia produced damage in 29.0±6.2% of CA1. In cultures treated with 7βOH-DHEA, a large, highly significant, reduction in neuronal damage was observed as shown in Table III, below.

**Table III**

| Compound | N | % Damage in CA1 |
|---|---|---|
| Control Hypoxia | 21 | 29.0±6.2 |
| Hypoxia + 100nM 7βOH-DHEA | 16 | 4.2±1.9** |

### EXAMPLE 22

### Global cerebral ischemia in rats (4 vessel occlusion)

Cerebral ischemia was induced by four-vessel-occlusion (4VO) in male Wistar rats (250-280g). Both vertebral arteries were occluded by electrocauterization in pentobarbital anesthesia (60 mg/kg i.p.). The animals were allowed to recover for 24 hours with free access to water but not food. The next day the carotid arteries were exposed under 2% halothane in 30% oxygen/70% nitrous oxide anesthesia and were occluded for 10 minutes using microvascular claps. Subsequently, both clamps were removed and both arteries were inspected for immediate reperfusion. During the operation and the following 3 hours normothermia of the animals (37.5±0.5°C) was maintained by using a thermostatically controlled heating blanket connected to a rectal thermometer. For control, in sham-operated animals both vertebral arteries were cauterized in pentobarbital anesthesia and both common carotid arteries were exposed but not clamped under 2% halothane in 30% oxygen/70% nitrous oxide anesthesia the following day. The wound was treated with lidocaine gel and then sutured. The animals were kept under a heating lamp at 30°C environmental temperature until they regained consciousness.

Seven groups of animals were investigated:
1. (n=8) steroid compound, 7β-OH EPIA (0.1 mg/kg, i. v. via tail vein, three injections: 15 minutes prior to the induction of ischemia, during ischemia and 5 minutes after reperfusion);
2. (n=8) steroid compound, 7β-OH EPIA (0.3 mg/kg, i. v. three injections as described in 1.);
3. (n=8) steroid compound, 7β-OH EPIA (1mg/kg, i. v., three injections as described in 1.);
4. (n=8) NBQX (disodium salt, because more water soluble) as reference substance and positive control (TOCRIS, Germany, 30mg/kg, i. p., three injections as described in 1.);
5. (n=8) received vehicle (0.9% NaCl, containing 100 µl Ethanol) three injections as described in 1.);
6. (n=8) ischemia alone;
7. (n=8) sham operated controls.

NBQX was 2,3-dihydroxy-6-nitro-7-sulfamoyl-benzo(F)quinoxaline and was known to have neuroprotective activity [Gill, R., Nordholm, L., Lodge D.: The neuroprotective action of 2,3-dihydroxy-6-nitro-7-sulfamoyl-benzo(F)quinoxaline (NBQX) in a rat focal ischaemia model. Brain Res. 580,35-43,1992].

7β-OH EPIA was 7β-hydroxyepiandrosterone, a compound of the present invention.

The substances were dissolved in 100 µl Ethanol and finally diluted with 0.9% NaCl.

After a survival time of 7 days after ischemia, all animals were perfusion fixed transcardially with 4% paraformaldehyde. The brains were then removed carefully and postfixed in the same fixative for 2 hours. After cryoprotection in 30% sucrose, the brains were rapidly frozen in isopentane and stored at -80°C. Twenty-micrometer cryostat sections comprising the hippocampal formation were Nissl stained with toluidine blue or NeuroTrace fluorescence.

### Data analysis:

The severity of neuronal damage in the hippocampal CA1 region after ischemia was evaluated by the number of surviving neurons using Nissl staining. The mean number of morphologically intact neurons per 400 µm length was calculated in CA1 region for each group. Cell counting was performed in 3-5 serial sections per animal and 6 times 400 µm CA1 area per section using a light microscope equipped with a 20 x objective. The data were statistically analyzed by paired Student's *t*-test. Data were presented as mean ± SEM.

### Results and Discussion

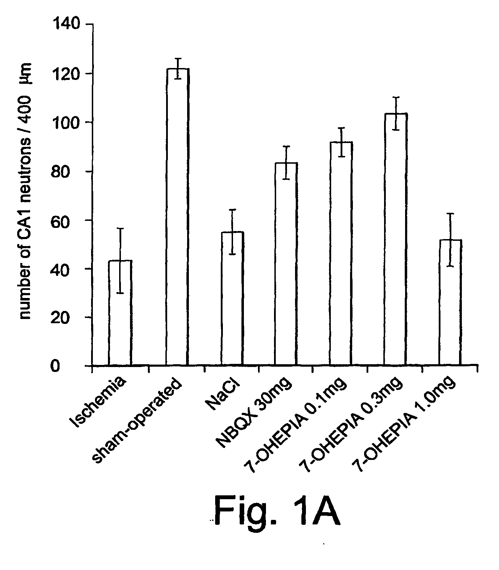

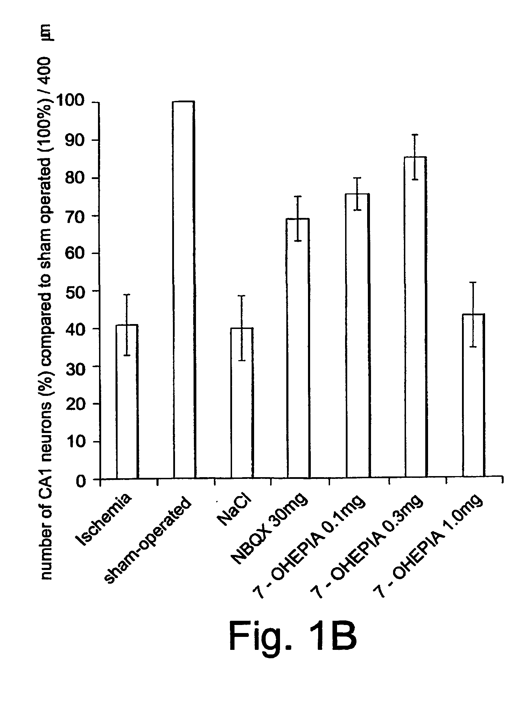

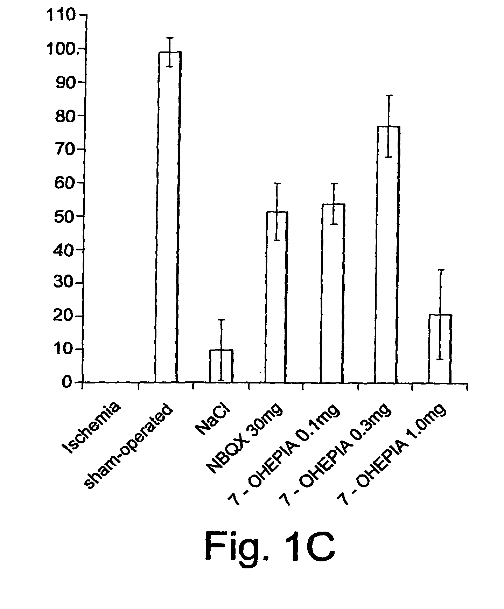

The results were shown in Figures 1 to 3 of the accompanying drawings.

Morphological intact hippocampal CA1 neurons were characterized by Nissl staining (toluidine blue and NeuroTrace, Fig. 2) with the following criteria: clear shape of a neuronal perikarya, large nucleus with a positive labeled nucleolus, a small cytoplasm zone around the nucleus with positive Nissl staining, indicating the intact rough endoplasmic reticulum with ribosomes and therefore the intact protein synthesis machinery.

10 minutes of global ischemia (mild ischemia) and a survival time of 7 days leads to a neurodegeneration of pyramidal cells selectively in the hippocampal CA1 region (Fig. 1A-1C). The mean number of pyramidal cells in CA1 of sham operated animals was 121.5±4.3 (set as 100%). Therefore, 60% of CA1 neurons died after 10 minutes of global ischemia (Fig. 1B). The number of neurons in the animal group of ischemia and i. v. injection of vehicle (NaCl plus 100 µl Ethanol) applied as described in the experiment was comparable to that of the ischemia group alone (Fig. 1A, 1B). NBQX (30 mg/kg, i.v., three injections as described in the experiment) showed a significant (p=0.03) neuroprotection in CA1 pyramidal cells compared to the ischemia group. Compared to the ischemia alone NBQX leads to a 47.5% neuroprotection while compared to the sham operated animals the protective effect was 68.5%. The neuroprotection caused by NBQX was in agreement with Gill et al., 1992 and Gill 1994 demonstrating the validity of the global ischemia model we used in our experiments. 7β-OH EPIA leads to a concentration dependent neuroprotection of hippocampal CA1 pyramidal cells after 10 minutes of global ischemia and a survival time of 7 days (Fig. 1A). T-test analysis revealed a highly significant neuroprotective effect of 7β-OH EPIA in concentrations of 0.1 mg/kg (p=0.01) and 0.3 mg/kg (p=0.0008). Compared to the sham operated group 7β-OH EPIA showed a 74.8% (0.1 mg/kg) and a 83.9% (0.3 mg/kg) neuroprotective effect on CA1 pyramidal cells, respectively (Fig. 1C). 7β-OH EPIA in a concentration of 1.0 mg/kg showed only a tendency to neuroprotection, but the effect was not significant.

In all experiments with 7β-OH EPIA injected i.v. prior, during and after ischemia we never observed any behavioral abnormalities of the animals.

### Legends of the Figures:

Number of morphological intact hippocampal CA1 pyramidal cells in rats 7 days after global cerebral ischemia in rats and under the influence of different compounds.
Fig. 1A: Data were presented as mean number ± SEM of intact neurons per 400 µm length of CA1 region.
Fig. 1B: Data were expressed as percentage of intact neurons per 400 µm length of CA1 region compared to sham operated animals set as 100%.
Fig. 1C: Data were presented as absolute percentage of neuroprotection when the number of surviving neurons in the ischemia group was set to zero and those of the sham operated group was set to 100%.

## Claims

1. The use for the manufacture of a medicament for protection against neuronal damage of a 7β-hydroxy steroid or its pharmaceutically acceptable ester or other derivative thereof, according to formula (I) or (II): 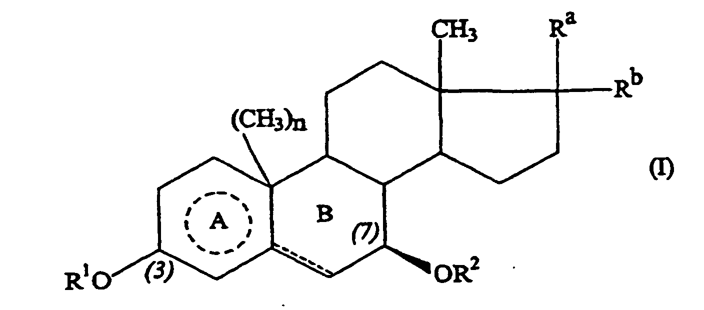 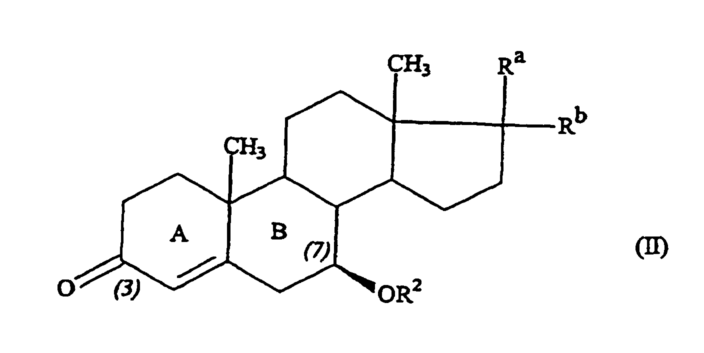 wherein
R¹ and R² are the same as or different from each other and each represents a hydrogen atom, an alkyl group having from 1 to 6 carbon atoms, an alkenyl group having from 2 to 6 carbon atoms, an alkynyl group having from 2 to 6 carbon atoms, an aryl group having from 6 to 10 carbon atoms, a formyl group, an alkylcarbonyl group having from 2 to 7 carbon atoms, an alkenylcarbonyl group having from 3 to 7 carbon atoms, an alkynylcarbonyl group having from 3 to 7 carbon atoms, an arylcarbonyl group having from 7 to 11 carbon atoms, an aralkylcarbonyl group having from 8 to 15 carbon atoms, an aralkenylcarbonyl group having from 9 to 15 carbon atoms, or a heterocyclic-carbonyl group, as defined below;
one of R^{a} and R^{b} represent a hydroxy group, preferably in the β configuration, and the other represents a hydrogen atom, or R^{a} and R^{b} together represent an oxo group;
the ring A, 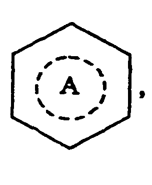 is a benzene or cyclohexane ring;
when ring A is a cyclohexane ring, the dotted line in ring B represents a single or double carbon-carbon bond and n is:1; or when ring A is a benzene ring, the dotted line in ring B represents a single carbon-carbon bond and n is 0;
said heterocyclic-carbonyl group is a group of formula R³-CO, where R³ represents a heterocyclic group having from 3 to 7 ring atoms, of which from 1 to 3 are hetero-atoms selected from nitrogen atoms, oxygen atoms and sulphur atoms, and the remaining atom or atoms of which there is at least one is or are carbon atoms;
said alkyl, alkenyl and alkynyl groups and the alkyl, alkenyl and alkynyl parts of said alkylcarbonyl, alkenylcarbonyl and alkynylcarbonyl groups being unsubstituted or having at least one of the following substituents ψ:
substituents ψ: hydroxy groups, mercapto groups, halogen atoms, amino groups, alkylamino groups having from 1 to 6 carbon atoms, dialkylamino groups in which each alkyl group has from 1 to 6 carbon atoms, carbamoyl groups, nitro groups, alkoxy groups having from 1 to 6 carbon atoms, alkylthio groups having from 1 to 6 carbon atoms, carboxy groups, alkoxycarbonyl groups and unsubstituted aryl groups having from 6 to 10 carbon atoms;
said aryl groups, said heterocyclic groups, and the aryl parts of said arylcarbonyl groups and said aralkylcarbonyl groups being unsubstituted or having at least one of the following substituents ξ:
substituents ξ: any of substituents ψ, and alkyl groups having from 1 to 6 carbon atoms, hydroxyalkyl groups having from 1 to 6 carbon atoms, and haloalkyl groups having from 1 to 6 carbon atoms;
and pharmaceutically acceptable salts and esters thereof.

2. The use according to Claim 1, in which:
R¹ and R² are the same as or different from each other and each represents a hydrogen atom, an alkyl group having from 1 to 6 carbon atoms, an optionally substituted phenyl group, a formyl group, an alkylcarbonyl group having from 2 to 5 carbon atoms, an arylcarbonyl group having from 7 to 11 carbon atoms, an aralkylcarbonyl group having from 8 to 15 carbon atoms, or a heterocyclic-carbonyl group, as defined below;
one of R^{a} and R^{b} represents a hydroxy group in the β configuration, and the other represents a hydrogen atom, or R^{a} and R^{b} together represent an oxo group;
said heterocyclic-carbonyl group is a group of formula R³-CO, where R³ represents a heterocyclic group having from 3 to 7 ring atoms, of which from 1 to 3 are hetero-atoms selected from nitrogen atoms, oxygen atoms and sulphur atoms, and the remaining atom or atoms of which there is at least one is or are carbon atoms.

3. The use according to Claim 1, in which the steroid is 7β-hydroxy-epiandrosterone.

4. The use according to Claim 1, in which the steroid is 7β-hydroxy-dehydroepiandrosterone.

5. The use according to Claim 1, in which the steroid is 7β-hydroxy-17β-oestradiol.

6. The use according to Claim 1, in which the steroid is 7β-hydroxy-oestrone.

7. The use according to Claim 1, in which the steroid is 7α-hydroxy-oestrone.

8. The use according to any one of the preceding Claims, in which the neuronal damage is caused by a chronic disorder.

9. The use according to Claim 8, in which the chronic disorder is Alzheimer's Disease, Parkinson's Disease, or Cognitive Impairment No Dementia.

10. The use according to any one of the preceding Claims, in which the neuronal damage is caused by an acute disorder.

11. The use according to Claim 10, in which the acute disorder is caused by stroke, brain trauma, spinal cord injury or peripheral nerve injury.

## Patentansprüche

1. Verwendung eines 7β-Hydroxysteroids oder eines pharmazeutisch akzeptierbaren Esters oder anderen Derivats davon gemäß Formel (I) oder (II) zur Herstellung eines Medikaments zum Schutz gegen Nervenschädigung, 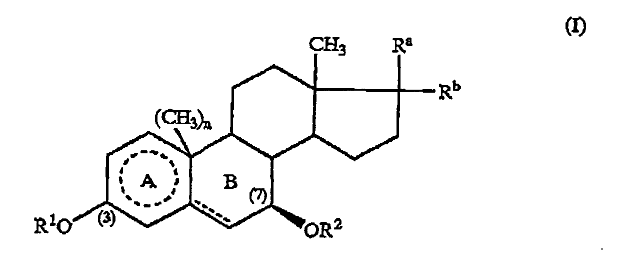 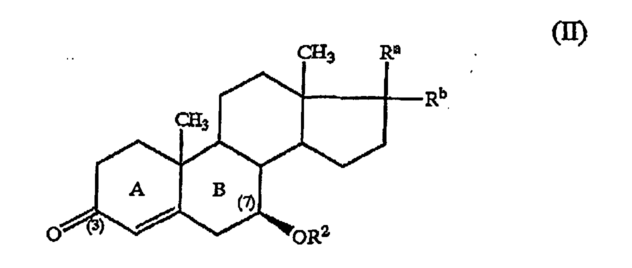 wobei R¹ und R² gleich oder voneinander verschieden sind und jeweils darstellen: ein Wasserstoffatom, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen, eine Arylgruppe mit 6 bis 10 Kohlenstoffatomen, eine Formylgruppe, eine Alkylcarbonylgruppe mit 2 bis 7 Kohlenstoffatomen, eine Alkenylcarbonylgruppe mit 3 bis 7 Kohlenstoffatomen, eine Alkinylcarbonylgruupe mit 3 bis 7 Kohlenstoffatomen, eine Arylcarbonylgruppe mit 7 bis 11 Kohlenstoffatomen, eine Aralkylcarbonylgruppe mit 8 bis 15 Kohlenstoffatomen, eine Aralkenylcarbonylgruppe mit 9 bis 15 Kohlenstoffatomen oder eine heterocyclische Carbonylgruppe gemäß der weiter unten gegebenen Definition;
wobei eine der Komponenten R^{a} und R^{b} eine Hydroxylgruppe darstellt, vorzugsweise in der β-Konfiguration, und die andere ein Wasserstoffatom darstellt, oder R^{a} und R^{b} zusammen eine Oxogruppe darstellen;
wobei der Ring A, 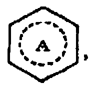 ein Benzol- oder Cyclohexanring ist;
wobei, wenn A ein Cyclohexanring ist, die gestrichelte Linie im Ring B eine Kohlenstoff-Kohlenstoff-Einfachbindung oder -Doppelbindung darstellt und n gleich 1 ist; oder wenn der Ring A ein Benzolring ist, die gestrichelte Linie im Ring B eine Kohlenstoff- Kohlenstoff-Einfachbindung darstellt und n gleich null ist;
wobei die heterocyclische Carbonylgruppe eine Gruppe mit der Formel R³-CO ist, wobei R³ eine heterocyclische Gruppe mit 3 bis 7 Ringatomen darstellt, von denen 1 bis 3 Heteroatome sind, die unter Stickstoffatomen, Sauerstoffatomen und Schwefelatomen ausgewählt sind, und wobei das übrige Atom oder die übrigen Atomen, wovon es mindestens eins gibt, Kohlenstoffatome sind;
wobei die Alkyl-, Alkenyl- und Alkinylgruppen und die Alkyl-, Alkenyl- und Alkinylteile der Alkylcarbonyl-, Alkenylcarbonyl- und Alkinylcarbonylgruppen nichtsubstituiert sind oder mindestens einen der folgenden Substituenten ψ aufweisen:
Substituenten ψ: Hydroxylgruppen, Mercaptogruppen, Halogenatome, Aminogruppen, Alkylaminogruppen mit 1 bis 6 Kohlenstoffatomen, Dialkylaminogruppen, in denen jede Alkylgruppe 1 bis 6 Kohlenstoffatome aufweist, Carbamoylgruppen, Nitrogruppen, Alkoxygruppen mit 1 bis 6 Kohlenstoffatomen, Alkylthiogruppen mit 1 bis 6 Kohlenstoffatomen, Carboxylgruppen, Alkoxycarbonylgruppen und nichtsubstituierte Arylgruppen mit 6 bis 10 Kohlenstoffatomen;
wobei die Arylgruppen, die heterocyclischen Gruppen und die Arylteile der Arylcarbonylgruppen und der Ara1kylcarbonylgruppen nichtsubstituiert sind oder mindestens einen der folgenden Substituenten ξ aufweisen:
Substituenten ξ: jeder der Substituenten ψ und Alkylgruppen mit 1 bis 8 Kohlenstoffatomen, Hydroxyalkylgruppen mit 1 bis 6 Kohlenstoffatomen und Halogenalkylgruppen mit 1 bis 6 Kohlenstoffatomen;
sowie von pharmazeutisch akzeptierbaren Salzen und Estern davon.

2. Verwendung gemäß Anspruch 1, wobei
R¹ und R² gleich oder voneinander verschieden sind und jeweils darstellen: ein Wasserstoffatom, eine Alkylgruppe mit 1 bis 6 Kohlenstoffatomen, eine wahlweise substituierte Phenylgruppe, eine Formylgruppe, eine Alkylcarbonylgruppe mit 2 bis 5 Kohlenstoffatomen, eine Arylcarbonylgruppe mit 7 bis 11 Kohlenstoffatomen, eine Aralkylearbonylgruppe mit 8 bis 15 Kohlenstoffatomen oder eine heterocyclische Carbonylgruppe gemäß der weiter unten gegebenen Definition;
wobei eine der Komponenten R^{a} und R^{b} eine Hydroxylgruppe in der β-Konfiguration darstellt und die andere ein Wasserstoffatom darstellt, oder R^{a} und R^{b} zusammen eine Oxogruppe darstellen;
wobei die heterocyclische Carbonylgruppe eine Gruppe mit der Formel R³-CO ist, wobei R³ eine heterocyclische Gruppe mit 3 bis 7 Ringatomen darstellt, von denen 1 bis 3 Heteroatome sind, die unter Stickstoffatomen, Sauerstoffatomen und Schwefelatomen ausgewählt sind, und wobei das übrige Atom oder die übrigen Atomen, wovon es mindestens eins gibt, Kohlenstoffatome sind.

3. Verwendung gemäß Anspruch 1, wobei das Steroid 7β-Hydroxyepiandrosteron ist.

4. Verwendung gemäß Anspruch 1, wobei das Steroid 7β-Hydroxydehydroepiandrosteron ist.

5. Verwendung gemäß Anspruch 1, wobei das Steroid 7β-Hydroxy-17β-astradiol ist

6. Verwendung gemäß Anspruch 1, wobei das Steroid 7β-Hydroxyöstron ist.

7. Verwendung gemäß Anspruch 1, wobei das Steroid 7α-Hydroxyöstron ist.

8. Verwendung nach einem der vorstehenden Ansprüche, wobei die Nervenschädigung durch eine chronische Erkrankung verursacht wird.

9. Verwendung gemäß Anspruch 8, wobei die chronische Erkrankung die Alzheimersche Krankheit, die Parkinsonsche Krankheit oder nichtdemenzielle Wahrnehmungsstörung ist.

10. Verwendung nach einem der vorstehenden Ansprüche, wobei die Nervenschädigung durch eine akute Erkrankung verursacht wird.

11. Verwendung gemäß Anspruch 10, wobei die akute Erkrankung durch Schlaganfall, Hirntrauma, Rückenmarkverletzung oder periphere Nervenverletzung verursacht wird.

## Revendications

1. Utilisation pour la production d'un médicament de protection contre les lésions neuronales d'un 7β-hydroxystéorïde ou de l'ester pharmaceutiquement acceptable correspondant ou d'un autre dérivé correspondant, selon la formule (I) ou (II): 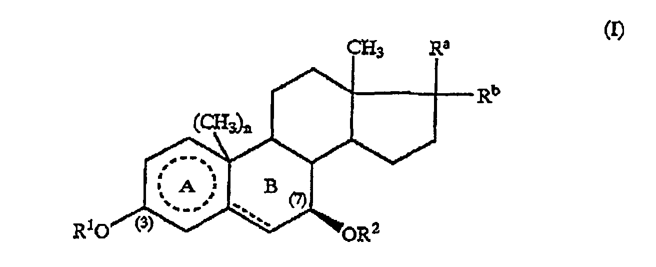 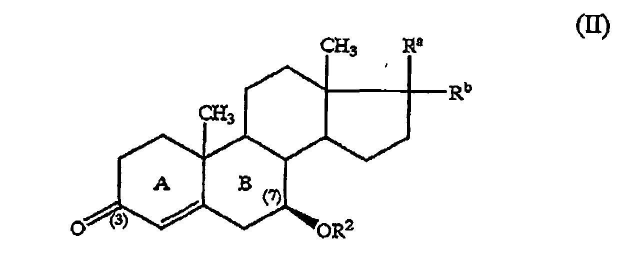 où
R¹ et R² sont identiques ou différents, chacun représentant un atome d'hydrogène, un groupe alkyle comportant 1 à 6 atomes de carbone, un groupe alkényle comportant 2 à 6 atomes de carbone, un groupe alkynyle comportant 2 à 6 atomes de carbone, un groupe aryle comportant 6 à 10 atomes de carbone, un groupe formyle, un groupe alkylcarbonyle comportant 2 à 7 atomes de carbone, un groupe alkénylcarbonyle comportant 3 à 7 atomes de carbone, un groupe alkynylcarbonyle comportant 3 à 7 atomes de carbone, un groupe arylcarbonyle comportant 7 à 11 atomes de carbone, un groupe aralkylcarbonyle comportant 8 à 15 atomes de carbone, un groupe aralkénylcarbonyle comportant 9 à 15 atomes de carbone, ou un groupe carbonyle hétérocyclique, comme défini ci-dessous;
un de R^{a} et R^{b} représentant un groupe hydroxy, de préférence dans la configuration β, et l'autre représentant un atome d'hydrogène, ou R^{a} et R^{b} ensemble représentent un groupe oxo;
le cycle A, 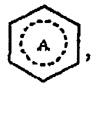 étant un cycle benzène ou cyclohexane;
lorsque le cycle A est un cycle cyclohexane, la ligne en pointillée dans le cycle B représente une liaison simple ou double carbone-carbone et n est 1; ou lorsque le cycle A est un cycle benzène, la ligne en pointillée dans le cycle B représente une liaison simple carbone-carbone et n est 0;
ledit groupe carbonyle hétérocyclique est un groupe de formule R³-CO, dans lequel R³ représente un groupe hétérocyclique possédant de 3 à 7 atomes cycliques, 1 à 3 desquels étant des hétéroatomes choisis parmi les atomes d'azote, d'oxygène et de soufre, l'atome ou les atomes restants, dont il y en a au moins un, est ou sont des atomes de carbone;
lesdits groupes alkyl, alkényl et alkynyl et les parties alkyles, alkényles et alkynyles desdits groupes alkylcarbonyles, alkénylcarbonyles et alkynylcarbonyles étant insubstitués ou ayant au moins l'un des substituants suivants Ψ;
substituants Ψ: groupes hydroxy, groupes mercapto, atomes d'halogène, des groupes amines, des groupes alkylamines ayant de 1 à 6 atomes de carbone, des groupes dialkylamines, dans lesquels chaque groupe alkyle a de 1 à 6 atomes de carbone, des groupes carbamoyles, des groupes nitro, des groupes alkoxy ayant de 1 à 6 atomes de carbone, des groupes alkylthio ayant de 1 à 6 atomes de carbone, des groupes carboxy, des groupes alkoxycarbonyles et des groupes aryles insubstitués ayant de 6 à 10 atomes de carbone;
lesdits groupes aryle, lesdits groupes hétérocycliques et les parts d'aryle desdits groupes arylcarbonyle et desdits groupes aralkylcarbonyle n'étant pas substitués ou comportant au moins un des substituants ξ suivants:
substituants ξ: un quelconque des substituants ψ, et des groupes alkyles comportant 1 à 6 atomes de carbone, des groupes hydroxyalkyles comportant 1 à 6 atomes de carbone, et des groupes haloalkyles comportant de 1 à 6 atomes de carbone;
et des sels ainsi que des esters correspondants, pharmaceutiquement acceptables.

2. Utilisation selon la revendication 1, dans laquelle:
R¹ et R² sont identiques ou différents, chacun représentant un atome d'hydrogène, un groupe alkyle comportant de 1 à 6 atomes de carbone, un groupe phényle à substitution optionnelle, un groupe formyle, un groupe alkylcarbonyle comportant 2 à 5 atomes de carbone, un groupe arylcarbonyle comportant 7 à 11 atomes de carbone, et un groupe aralkylcarbonyle comportant 8 à 15 atomes de carbone ou un groupe carbonyle hétérocyclique, comme défini ci-dessous;
un de R^{a} ou R^{b} représentant un groupe hydroxy dans la configuration β, l'autre représentant un atome d'hydrogène, ou R^{a} et R^{b} représentant ensemble un groupe oxo;
ledit groupe carbonyle hétérocyclique étant un groupe de formule R³-CO; où R³ représente un groupe hétérocyclique comportant 3 à 7 atomes cycliques, 1 à 3 desquels étant des hétéroatomes sélectionnés parmi les atomes d'azote, les atomes d'oxygène et les atomes de soufre, l'atome ou les atomes restants, dont il y en a au moins un, est ou sont des atomes de carbone.

3. Utilisation selon la revendication 1, dans laquelle le stéroïde est un 7β-hydroxy-épiandrostérone.

4. Utilisation selon la revendication 1, dans laquelle le stéroïde est un 7β-hydroxy-déhydro-épiandrostérone.

5. Utilisation selon la revendication 1, dans laquelle le stéroïde est un 7β-hydroxy-17β-oestradiol.

6. Utilisation selon la revendication 1, dans laquelle le stéroïde est un 7β-hydroxy-oestrone.

7. Utilisation selon la revendication 1, dans laquelle le stéroïde est un 7α-hydroxy-oestrone.

8. Utilisation selon l'une quelconque des revendications précédentes, dans laquelle la lésion neuronale est causée par un désordre chronique.

9. Utilisation selon la revendication 8, dans laquelle le désordre chronique est une maladie d'Alzheimer, une maladie de Parkinson, ou une déficience intellectuelle non liée à une démence.

10. Utilisation selon l'une quelconque des revendications précédentes, dans laquelle la lésion neuronale est causée par un désordre aigu.

11. Utilisation selon la revendication 10, dans laquelle le désordre aigu est causé par un accident vasculaire cérébral, un tmumatisme cérébral, une lésion de la moelle épinière ou une lésion du nerf périphérique.
